# FASCICULE DE BREVET EUROPEEN

(11) **EP 2 533 756 B1**
(45) Date de publication et mention de la délivrance du brevet: **17.08.2016**
(21) Numéro de dépôt: 11702266.5
(22) Date de dépôt: 09.02.2011
(51) Int. Cl.: A61K 8/37, A61Q 5/02, A61K 8/41, A61K 8/46, A61K 8/49, A61K 8/92

(54) **COMPOSITION POUR CHEVEUX CREPUS ET/OU TRES SECS**
ZUSAMMENSETZUNG FÜR KRÄUSELIGE UND/ODER TROCKENE HAARE
COMPOSITION FOR FRIZZY AND/OR DRY HAIR

(30) Priorité: 09.02.2010 FR 1050887
(43) Date de publication de la demande: 19.12.2012
(73) Titulaire: Pierre Fabre Dermo-Cosmétique, 92100 Boulogne-Billancourt (FR)
(72) Inventeur: BACO, David, F-31860 Labarthe Sur Leze (FR)
(74) Mandataire: Regimbeau
(86) Numéro de dépôt international: PCT/EP2011/051886
(87) Numéro de publication internationale: WO 2011/098481

(56) Documents cités:
- EP-A2- 1 604 641
- EP-B1- 1 165 019
- EP-B1- 1 465 584
- WO-A1-2006/023591
- WO-A2-2007/132273
- FR-A1- 2 700 954
- "Cosmetic Color Variable Pigment Formulations", IP.COM JOURNAL, IP.COM INC., WEST HENRIETTA, NY, US, 28 octobre 2009 (2009-10-28), XP013134989, ISSN: 1533-0001

## Description

La présente invention concerne un shampoing capillaire comprenant une phase grasse, un mélange de tensioactifs et un composé cationique comprenant au moins un composé soit de nature polymérique soit de nature tensioactive pour le lavage et le soin des cheveux crépus et/ou très secs.

Le cheveu crépu concerne en particulier les populations noires d'origine africaine. Ce type de cheveu présente une physiologie et un comportement très atypiques et totalement différents du cheveu caucasien.

La particularité essentielle du cheveu crépu est d'avoir une croissance en torsion hélicoïdale en présentant une section elliptique et non arrondie. Ces deux caractéristiques sont la cause de l'aspect crépu, et expliquent également la pousse lente de ce type de cheveu (moins de 1 centimètre/mois) et de sa tendance à l'emmêlement.

L'aspect du cheveu crépu est sans doute expliqué par l'incurvation des follicules pileux, déterminant sa pousse hélicoïdale et un agencement particulier des ponts disulfures entre les molécules de cystéine.

La sécrétion sébacée est faible et détermine une sécheresse importante des cheveux crépus.

Les cheveux crépus sont donc moins élastiques et plus cassants. Leur configuration torsadée les rend plus fragiles et plus difficiles à coiffer.

La fragilité des cheveux crépus est due à la présence de zones d'écrasement qui constituent des points de faiblesse mécanique où la rupture est facile, d'autant plus que ce type de cheveu absorbe moins d'eau indispensable au maintien des propriétés élastiques de la kératine.

La répartition des lipides diffère également d'un type de cheveu à un autre. En effet, pour les cheveux de type caucasien, les lipides sont majoritairement localisés dans la médula à savoir la partie centrale du cheveu tandis que dans le cas des cheveux crépus, les lipides se situent majoritairement dans la zone corticale à savoir la partie externe du cheveu.

De la même façon en ce qui concerne la cuticule, qui constitue la partie gainante externe du cheveu il existe des différences entre les différents types de cheveu. Dans le cas du cheveu caucasien, la cuticule est relativement homogène tandis que dans le cas du cheveu crépu, la cuticule présente une structure très irrégulière. Les cellules externes cuticulaires sont donc le plus souvent endommagées ce qui accroît la fragilité du cheveu crépu.

A cette fragilité inhérente au cheveu crépu, il faut également ajouter les différents traitements dont ils font l'objet pour répondre à des exigences esthétiques.

En effet, pour diminuer cet aspect crépu et améliorer la tendance à l'emmêlement, le défrisage ou le tressage des cheveux sont fréquemment utilisés.

Effectivement, 80% des femmes d'origine africaine défrisent leurs cheveux toutes les 4 à 6 semaines.

Le défrisage permet de décrêper le cheveu et de lui donner du mouvement. Le défrisage peut être pratiqué à chaud à l'aide de fers ce qui constitue une technique très traumatisante. Les défrisages chimiques aux alcalis ou à l'acide thioglycolique sont moins agressifs que la méthode précédente mais néanmoins fragilisent la tige capillaire induisant des ruptures, voire des chutes de cheveux.

Ces pratiques capillaires peuvent causer des dénaturations capillaires pouvant aller jusqu'à des atteintes chimiques et structurales irréversibles de la tige kératinique.

Pour l'ensemble de ces raisons, le cheveu crépu nécessite des soins adaptés.

Le marché actuel propose aux consommateurs en majorité des produits de soins capillaires à utiliser après le lavage des cheveux, mais peu de produits d'hygiène permettant d'associer lavage du cheveu et soin spécifiquement adapté aux cheveux crépus et/ou très secs.

D'un point de vue pathologique, des cheveux deviennent très secs suite à une défaillance de fonctionnement de la glande sébacée. Ceci entraîne une diminution de la production de sébum, et par conséquent une moins bonne protection et une fragilisation du cheveu. Par cheveux très secs, on entend des cheveux devenus fragiles et cassants suite aux différents traitements tels que permanentes, lissages et brushings.

L'état de la technique antérieure peut par exemple être illustré par la demande WO2006/023591 qui décrit des compositions à base de sphérulite délivrant une phase huileuse constituée par une huile, seule l'huile de tournesol étant exemplifiée.

La présente invention concerne donc un shampoing capillaire permettant le lavage tout en associant un soin spécifique permettant le démêlage, la nutrition et la réparation des cheveux crépus et/ou très secs.

L'objet de la présente invention est un shampoing capillaire pour cheveux crépus et/ou très secs comprenant :
1% à 30 % en poids d'une phase grasse comprenant de l'huile de crambe d'Abyssinie,
10 % à 60 % en poids d'un mélange de tensioactifs comprenant du sodium trideceth sulfate et du sodium lauroamphoacetate, et
0,01 % à 10 % en poids d'un composé cationique comprenant au moins un composé soit de nature polymérique soit de nature tensioactive.

Par phase grasse, on entend les composés lipophiles comme les huiles, les gommes, les pâtes et les cires ou beurres.

Les huiles peuvent être choisies parmi les huiles végétales, les huiles animales, les huiles minérales, les huiles de synthèse, les huiles siliconées, les esters d'acides gras liquides, les acides gras liquides et les amides gras liquides.

Comme huile minérale, on peut citer les huiles de paraffines et les silicones

Comme huile végétale, on peut notamment mentionner l'huile d'amande douce, l'huile d'avocat, l'huile de ricin, l'huile d'olive, la cire liquide jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de noyau d'abricot et l'huile de calophyllum, l'huile de karité, l'huile de crambe d'Abyssinie.

Comme huile animale, on peut notamment citer le perhydrosqualène.

Comme huile de synthèse, on peut citer le squalane, les poly (α-oléfines) comme l'isododécane ou l'isohexadécane, les huiles végétales transestérifiées et les huiles fluorées.

Comme huiles siliconées, on peut citer les polydiméthylsiloxanes cycliques (nom INCI : cyclométhicone) tels que le décaméthyl pentasiloxane et les polyméthylsiloxanes linéaires de faible viscosité.

Les cires ou beurres utilisables dans la présente invention sont par exemple les cires ou beurres d'origine animale, d'origine végétale, minérale ou synthétique telle que la cire d'abeille, le spermaceti, les cires fluorées ou perfluorées, les cires de lanoline, les cires de Candellila, d'Ouricury, de Carnauba, du Japon, de beurre de cacao, le beurre de karité, le beurre d'illipé, le beurre de mangue, les cires de fibres de liège ou de canne à sucre, la cire de son de riz, la cire de sapin, la cire de coton ; les cires microcristallines, la cire de paraffine, le petrolatum, la vaseline, l'ozokérite, la cire de montan ; les huiles hydrogénées ayant une température supérieure à 40°C comme l'huile de jojoba hydrogénée, les cires de polyéthylène et les cires obtenues par synthèse de Fischer - Tropsch.

Les cires peuvent également être choisies parmi les alcools gras cireux et esters gras cireux.

Les esters d'acides gras cireux sont des esters d'acides gras, c'est-à-dire des esters d'acides carboxyliques comportant au moins 10 atomes de carbone et d'un monoalcool ou d'un polyol. Les esters d'acides gras cireux utilisables dans le shampoing selon l'invention peuvent être des mono, des di, ou des triesters. A titre d'exemples d'esters cireux, on peut citer le myristate de myristyle et le stéarate de stéaryle.

Les acides gras cireux utilisables dans le shampoing selon l'invention comportent de préférence de 12 à 24 atomes de carbone et peuvent être saturés ou insaturés, ramifiés ou non, et comporter une ou plusieurs fonctions hydroxy. On peut citer par exemple, l'acide laurique, l'acide stéarylique, l'acide cétylique et l'acide béhénique.

A titre d'amides cireux, on peut mentionner les céramides comme par exemple la noléyldihydrosphingosine.

Pour la réalisation de l'invention les triglycérides d'origine végétale (C₈-C₂₂) seront privilégiés, ils peuvent être sélectionnés parmi les huiles et les beurres et on peut en particulier citer : l'huile de carthame, l'huile de sésame, l'huile de tournesol, l'huile d'avocat, l'huile et le beurre de karité, le beurre d'illipé, le beurre de mangue, l'huile d'olive, l'huile de crambe d'Abyssinie.

Dans un mode de réalisation de l'invention la phase grasse comprend un ou plusieurs triglycéride(s) d'origine végétale (C₈-C₂₂).

Dans un autre mode de réalisation de l'invention, la phase grasse du shampoing capillaire est constituée uniquement d'un ou plusieurs triglycéride(s) d'origine végétale.

La phase grasse shampoing capillaire comprend de l'huile de crambe d'Abyssinie (INCI : Crambe Abyssinia seed oil).

Il s'agit d'une petite graine de moutarde issue de la famille des brassicacée qui fournit une huile particulièrement riche en triglycérides de longues chaînes. En effet, sa teneur en acide érucique est supérieure à 50%. Liquide à température ambiante, sa composition en fait une véritable cire liquide pouvant compenser le déficit de sébum des cheveux crépus et/ou très secs. Cette huile possède aussi la particularité d'être très stable à la chaleur et à l'oxydation.

Dans un mode de réalisation de l'invention, la phase grasse du shampoing capillaire est constituée d'huile de crambe d'Abyssinie en association avec un ou plusieurs triglycéride(s) d'origine végétale.

Selon l'invention, le shampoing capillaire est caractérisé en ce qu'il comprend de 1% à 30% en poids et de préférence de 5% à 20% en poids de phase grasse par rapport au poids total du shampoing.

Le shampoing capillaire peut comprendre en outre un polyol miscible à l'eau à la température ambiante (25 °C) notamment choisi parmi les polyols ayant notamment de 2 à 20 atomes de carbones, de préférence ayant de 2 à 10 atomes de carbone, et préférentiellement ayant de 2 à 6 atomes de carbones, tels que le glycérol, les dérivés de glycol tel que le propylène glycol, le butylène glycol, le pentylène glycol, l'hexylène glycol, le dipropylène glycol, le diéthylène glycol ; les éthers de glycol tels que les alkyl(C₁-C₄)éther de mono, di- ou tripropylène glycol, les alkyl(C₁-C₄)éthers de mono, di- ou triéthylène glycol ; et leurs mélanges.

Selon un mode de réalisation de l'invention, le shampoing capillaire comprend de 1% à 10% de polyol miscible à l'eau par exemple, le glycérol.

Selon un mode particulier de réalisation de l'invention, le shampoing capillaire est caractérisé en ce que le mélange de tensioactifs est constitué de 35 à 40% de sodium trideceth sulfate et de 10 à 15% de sodium lauroamphoacetate et de 45% à 55% d'eau.

En particulier, le mélange de tensioactifs est constitué en moyenne de 38% de sodium trideceth sulfate et de 12% de sodium lauroamphoacetate et de 50% d'eau.

Le mélange de tensioactifs selon l'invention peut être le mélange de tensioactifs Miracare SLB®, en particulier Miracare SLB 205N® développé par la société Rhodia. Cette base tensioactive permet d'introduire de grandes quantités de phase grasse (1 à 20%) telles que les huiles végétales, esters, huiles minérales et silicone tout en conservant d'excellentes qualités cosmétiques (qualité et quantité de mousse, pouvoir nettoyant du cheveu et du cuir chevelu).

De façon préférée, le shampoing capillaire selon l'invention est caractérisé en ce qu'il comprend de 10% à 60% en poids et de préférence de 20% à 50% en poids de mélange de tensioactifs par rapport au poids total du shampoing.

Le shampoing capillaire selon l'invention comprend également un composé cationique comprenant au moins un composé soit de nature polymérique soit de nature tensioactive. Ces composés cationiques confèrent des propriétés cosmétiques comme le démêlage, l'hydratation, la douceur et la brillance.

Selon un mode de réalisation de l'invention, le composé cationique comprenant au moins un composé soit de nature polymérique soit de nature tensioactive est sélectionné parmi le groupe constitué par : le chlorure de béhentrimonium, les polyquaterniums, les ammoniums quaternaires de chaînes alkyles comprises entre C₁₂ et C₂₂, les polymères de guar quaternisées, les protéines quaternisées, le chlorure de cétrimomium et le complexe contenant du chlorure d'acrylamidopropyltrimonium et du copolymère d'acrylamide.

En particulier, le shampoing capillaire est caractérisé en ce que le composé cationique comprenant au moins un composé soit de nature polymérique soit de nature tensioactive est sélectionné parmi le groupe constitué par : le chlorure de béhentrimonium, le polymère de guar quaternisée et le chlorure de cétrimomium.

De façon préférée, le shampoing capillaire selon l'invention est caractérisé en ce qu'il comprend les composés cationiques comprenant au moins un composé soit de nature polymérique soit de nature tensioactive suivants : le chlorure de béhentrimonium, le polymère de guar quaternisée et le chlorure de cétrimomium.

Selon un mode de réalisation particulier de l'invention, le shampoing capillaire est caractérisé en ce qu'il comprend 0,01% à 10% en poids et de préférence de 0,1% à 5% en poids de composé (s) cationique (s) comprenant au moins un composé soit de nature polymérique soit de nature tensioactive par rapport au poids total de la composition.

Selon l'invention, le shampoing capillaire comprend en outre un support cosmétiquement acceptable. Par « support cosmétiquement acceptable », on entend tout adjuvant ou excipient permettant la fabrication, la conservation ou l'administration topique du shampoing capillaire.

Les shampoings selon l'invention peuvent comprendre en outre des adjuvants cosmétiques classiques notamment choisis parmi les corps gras, les solvants organiques, les épaississants, les adoucissants, les opacifiants, les stabilisants, les émollients, les agents hydratants, les parfums, les conservateurs, les polymères, les charges, les séquestrants, les bactéricides et/ou les absorbeurs d'odeur, les agents alcalinisants ou acidifiants, les tensio-actifs, les anti-radicaux libres, les antioxydants, les vitamines E et C, les α-hydroxyacides ou tout autre ingrédient habituellement utilisé en cosmétique, en particulier pour la fabrication des shampoings.

Selon un mode de réalisation préféré, l'utilisation du shampoing capillaire selon l'invention est destinée au traitement capillaire des cheveux crépus et/ou très secs.

L'objet de la présente invention comprend également un procédé de lavage des cheveux crépus et/ou très secs comprenant l'application sur la chevelure d'un shampoing capillaire tel que décrit précédemment.

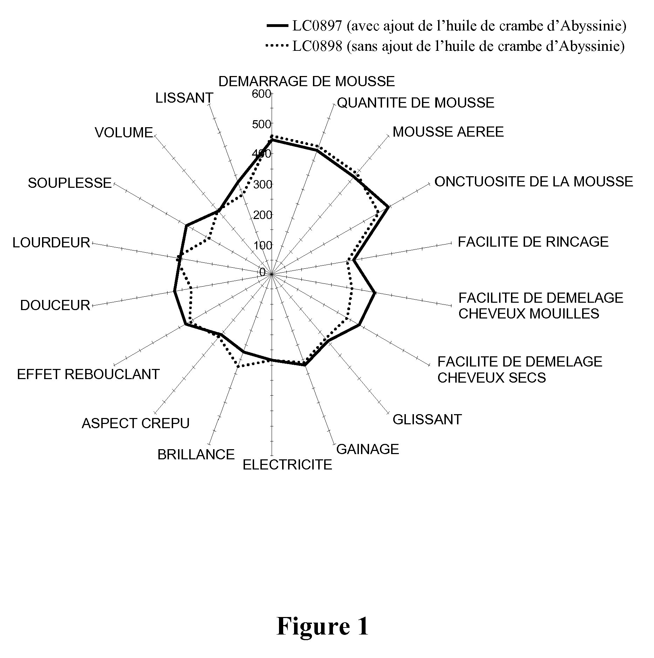

La description se réfère à la Figure 1 représentant un graphique « en radar » comparant les résultats obtenus après l'utilisation d'un shampoing capillaire comprenant de l'huile de crambe d'Abyssinie (courbe pleine) et ne comprenant pas de l'huile de crambe d'Abyssinie (courbe en pointillés).

### - Evaluation de l'efficacité du shampoing capillaire par test d'usage consommateurs

Cette étude avait pour objectifs de vérifier la tolérance cutanée sous contrôle dermatologique du shampooing selon l'invention ainsi que ses qualités cosmétiques, après utilisation à domicile pendant 2 semaines consécutives, dans les conditions normales d'emploi. Cette étude a été réalisée sur 32 volontaires. Les critères de sélection des volontaires étaient les suivants :
- Age : au minimum 18 ans
- Sexe : féminin
- Phototype VI (origine africaine)
- Type de cheveux : Cheveux très secs et crépus
- Utilisatrices habituelles de shampoings destinés à cette cible

**- Formule du shampoing selon l'invention testé :**

| | | |
|---|---|---|
| Miracare SLB 205 N® | → | 40 % |
| Huile de crambe d'Abyssinie | → | 5% |
| Triglycérides d'origine végétale (C₈-C₂₂) | → | 10% |
| Glycérol | → | 5 % |
| Chlorure de hydroxypropyltrimonium de guar | → | 0.2 % |
| Chlorure de béhentrimonium | → | 2% |
| Chlorure de cétrimonium | → | 0.6 % |
| Chlorure de sodium | → | QSP |
| Agents conservateurs | → | QSP |
| Fragrance | → | 0,5% |
| Acide citrique | → | QSP pH = 5.5 |
| Eau déminéralisée | → | QSP 100 |

### - Application du produit - Fréquence - Modalités

Les indications d'utilisation étaient les suivantes :
« Sur cheveux mouillés, appliquer le shampooing en massant délicatement le cuir chevelu et les cheveux. Un seul shampooing suffit. Rincer longuement à l'eau claire. En cas de contact avec les yeux, rincer à l'eau claire. »

Le shampoing selon l'invention devait être utilisé au moins 2 fois par semaine, pendant 2 semaines consécutives.

A l'issue de cette période d'utilisation, les volontaires ont répondu à un questionnaire permettant d'évaluer les qualités cosmétiques ainsi que l'efficacité du shampoing selon l'invention en comparaison avec leur shampoing habituel.

**- Qualités cosmétiques et techniques**

| | |
|---|---|
| Aspect attrayant | 94 % |
| Couleur plaisante | 97 % |
| Consistance satisfaisante | 94 % |
| Application facile | 94 % |
| Parfum agréable | 91 % |
| Intensité du parfum satisfaisante | 88 % |
| Temps de développement de la mousse satisfaisant | 84 % |
| Mousse abondante | 81 % |
| Mousse onctueuse | 84 % |
| Rinçage facile | 97 % |
| Facilité de démêlage cheveux mouillés | 84 %* |
| Facilité de démêlage cheveux secs | 85 %** |

| | |
|---|---|
| * Base : 31 femmes (1 non concernée) ** Base : 27 femmes (5 non concernées) | |

L'ensemble des qualités cosmétiques du produit ont été appréciées par la majorité des volontaires.

**- Etat des cheveux après application**

| | |
|---|---|
| Etat des cheveux satisfaisant | 84 % |
| Doux | 81 % |
| Souples | 84 % |
| Brillants | 59 % |
| Faciles à coiffer | 81 % |
| Avec de la tenue | 69 % |
| Nourris | 69 % (+ oui un peu : 88 %) |
| Hydratés | 75 % (+ oui un peu : 88 %) |
| Protégés de la déshydratation | 75 % (+ oui un peu : 81 %) |
| Non gras | 97 % |
| Non électriques | 94 % |
| Non rêches | 88 % |
| Non secs | 75 % |
| Non alourdis | 84 % |
| Non ternes | 91 % |

Après utilisation du shampooing selon l'invention plus de 8 volontaires sur 10 jugent l'état de leurs cheveux satisfaisant. Les cheveux sont doux, souples, faciles à coiffer. Les volontaires considèrent pour une grande majorité que le produit nourrit et protège de la déshydratation sans graisser ni alourdir la chevelure.

### - Efficacité perçue après 2 semaines d'utilisation

Après 2 semaines d'utilisation, les volontaires considèrent que leurs cheveux sont :
□ restructurés : 81%*
□ nourris : 88%*
□ plus doux : 88%*
□ plus souples : 88%*
□ protégés de la déshydratation : 81%*
□ plus faciles à coiffer : 87%*
□ Facilité de démêlage : 85%*
□ Eclat : 88%*
* : cumul des résultats « oui tout à fait » + « oui » + « oui, un peu » (avec « oui tout à fait » + « oui » >50%) obtenus dans le test d'usage sous contrôle dermatologique

Au terme de 15 jours d'utilisation, 88% des volontaires étaient satisfaites du pouvoir nutritif du shampooing. De plus, le shampoing testé selon l'invention présente une bonne tolérance cutanée après utilisation pendant 2 semaines consécutives chez des volontaires présentant tous types de peau, un cuir chevelu sec et des cheveux très secs et crépus.

### - Evaluation de l'efficacité de l'ajout de l'huile de crambe d'Abyssinie au shampoing capillaire par test d'usage consommateurs

Cette étude avait pour objectifs de comparer les qualités cosmétiques et techniques obtenues après l'utilisation de deux shampoings comprenant soit un shampoing capillaire avec ajout de l'huile de crambe d'Abyssinie (LC0897) soit un shampoing capillaire sans ajout de l'huile de crambe d'Abyssinie (LC0898).

Cette étude a été réalisée sur 12 volontaires. Le protocole d'utilisation du shampoing est identique à celui décrit dans l'étude précédente. Les volontaires ont répondu à un questionnaire, leurs réponses ont été exprimées de façon identique à l'étude précédente puis traduites en unités arbitraires de manière à pouvoir exprimer toutes les données sur un même graphique (Figure 1).

**- Formule des shampoings testés selon l'invention:**

| | Composition (%) | |
|---|---|---|
| Ingrédient | LC0897 | LC0898 |
| | | |
| Acide citrique monohydraté | 0.82 | 0.82 |
| Triglycérides capryliques/capriques | 10 | 10 |
| Glycérol 99.5% | 5 | 5 |
| Chlorure de sodium | 3.5 | 3.5 |
| Chlorure de hydroxypropyltrimonium de guar | 0.2 | 0.2 |
| Eau purifiée | 34.43 | 39.43 |
| Parfum | 0.5 | 0.5 |
| Huile de crambe d'Abyssinie | 5 | 0 |
| Lécithine | 0.5 | 0.5 |
| Méthylisothiazolinone | 0.05 | 0.05 |
| Miracare SLB 205 N® | 40 | 40 |

### - Etat des cheveux après applications

La figure 1 montre que l'ajout de l'huile de crambe d'Abyssinie au shampoing capillaire permet d'obtenir une mousse plus onctueuse et d'augmenter significativement la douceur, la souplesse et l'aspect lissant du cheveu. Les cheveux sont également beaucoup plus faciles à démêler.

### - Exemples de shampoings capillaires selon l'invention :

Ces exemples sont cités à titre illustratif et ne limitent en rien la portée de l'invention.

**- shampoing crème pour cheveux très secs et cassants**

| | | |
|---|---|---|
| Miracare SLB 205 N® | → | 40 % |
| Triglycérides d'origine végétale (C₈-C₂₂) | → | 15 % |
| Glycérol | → | 5 % |

Salcare SC 60® (complexe contenant
du chlorure d'acrylamidopropyltrimonium et

| | | |
|---|---|---|
| du copolymère d'acrylamide) | → | 0,5 % |
| Chlorure de sodium | → | QSP |
| Agents conservateurs | → | QSP |
| Acide citrique | → | QSP pH = 5.5 |
| Eau déminéralisée | → | QSP 100 |

**- shampoing pour cheveux crépus**

| | | |
|---|---|---|
| Miracare SLB 205 N® | → | 40 % |
| Huile de crambe d'Abyssinie | → | 7 % |
| Huile d'avocat | → | 8 % |
| Glycérol | → | 5 % |
| Salcare SC 60® | → | 0.5 % |
| Chlorure de sodium | → | QSP |
| Fragrance | → | QSP |
| Conservateurs | → | QSP |

**- shampoing réparateur ultra nutritif**

| | | |
|---|---|---|
| Miracare SLB 205 N® | → | 40 % |
| Triglycéride de Karité | → | 5 % |
| Huile de crambe d'Abyssinie | → | 10 % |
| Chlorure de hydroxypropyltrimonium de guar | → | 0.3 % |
| Chlorure de béhentrimonium | → | 2 % |
| Chlorure de cétrimonium | → | 0.6 % |
| Chlorure de sodium | → | QSP |
| Phospholipides de soja | → | 0.5 % |
| Fragrance | → | QSP |
| Eau | → | QSP 100 |

## Revendications

1. Shampoing capillaire pour cheveux crépus et/ou très secs comprenant :
- 1% à 30 % en poids d'une phase grasse comprenant de l'huile de crambe d'Abyssinie,
- 10 % à 60 % en poids d'un mélange de tensioactifs comprenant du sodium trideceth sulfate et du sodium lauroamphoacetate, et
- 0,01 % à 10 % en poids d'un composé cationique comprenant au moins un composé soit de nature polymérique soit de nature tensioactive.

2. Shampoing capillaire pour cheveux crépus et/ou très secs selon la revendication 1, **caractérisé en ce que** la phase grasse comprend une association d'huile de crambe d'Abyssinie avec un ou plusieurs triglycéride(s) d'origine végétale.

3. Shampoing capillaire pour cheveux crépus et/ou très secs selon les revendications 1 à 2, **caractérisé en ce qu'**il comprend de 5% à 20% en poids de phase grasse par rapport au poids total du shampoing.

4. Shampoing capillaire pour cheveux crépus et/ou très secs selon l'une des revendications 1 à 3, **caractérisé en ce que** le mélange de tensioactifs est constitué de 35 à 40% de sodium trideceth sulfate et de 10% à 15% de sodium lauroamphoacétate et 45% à 55% d'eau.

5. Shampoing capillaire pour cheveux crépus et/ou très secs selon l'une des revendications 1 à 4, **caractérisé en ce que** le mélange de tensioactifs est constitué de 38% de sodium trideceth sulfate et de 12% de sodium lauroamphoacetate et de 50% d'eau.

6. Shampoing capillaire pour cheveux crépus et/ou très secs selon l'une des revendications 1 à 5, **caractérisé en ce qu'**il comprend de 20% à 50% en poids de mélange de tensioactifs par rapport au poids total du shampoing.

7. Shampoing capillaire pour cheveux crépus et/ou très secs selon l'une des revendications 1 à 6, **caractérisé en ce que** le composé cationique comprenant au moins un composé, soit de nature polymérique soit de nature tensioactive, est sélectionné parmi le groupe constitué par : le chlorure de béhentrimonium, les polyquaterniums, les ammoniums quaternaires de chaînes alkyles comprises entre C12 et C22, les polymères de guar quaternisées, les protéines quaternisées, le chlorure de cétrimomium et le complexe contenant du chlorure d'acrylamidopropyltrimonium et du copolymère d'acrylamide.

8. Shampoing capillaire pour cheveux crépus et/ou très secs selon l'une des revendications 1 à 7, **caractérisé en ce que** le composé cationique comprenant au moins un composé, soit de nature polymérique soit de nature tensioactive, est sélectionné parmi le groupe constitué par : le chlorure de béhentrimonium, le polymère de guar quaternisée et le chlorure de cétrimomium.

9. Shampoing capillaire pour cheveux crépus et/ou très secs selon l'une des revendications 1 à 8, **caractérisé en ce qu'**il comprend de 0,1% à 5% en poids de composé (s) cationique (s) comprenant au moins un composé, soit de nature polymérique soit de nature tensioactive, par rapport au poids total du shampoing.

10. Procédé de lavage des cheveux crépus et/ou très secs comprenant l'application sur la chevelure d'un shampoing capillaire pour cheveux crépus et/ou très secs selon les revendications 1 à 9.

## Patentansprüche

1. Haarshampoo für krauses und/oder sehr trockenes Haar, umfassend:
- 1 bis 30 Gew.-% einer Fettphase, umfassend abessinisches Krambeöl,
- 10 bis 60 Gew.-% einer Mischung aus oberflächenaktiven Stoffen, umfassend Natriumtridecethsulfat und Natriumlauroamphoacetat, und
- 0,01 bis 10 Gew.-% einer kationischen Verbindung, umfassend mindestens eine Verbindung entweder polymerer Art oder oberflächenaktiver Art.

2. Haarshampoo für krauses und/oder sehr trockenes Haar nach Anspruch 1, **dadurch gekennzeichnet, dass** die Fettphase eine Assoziation aus abessinischem Krambeöl mit einem oder mit mehreren Triglycerid(en) pflanzlichen Ursprungs umfasst.

3. Haarshampoo für krauses und/oder sehr trockenes Haar nach Anspruch 1 bis 2, **dadurch gekennzeichnet, dass** es von 5 bis 20 Gew.-% Fettphase mit Bezug auf das Gesamtgewicht des Shampoos umfasst.

4. Haarshampoo für krauses und/oder sehr trockenes Haar nach einem der Ansprüche 1 bis 3, **dadurch gekennzeichnet, dass** die Mischung aus oberflächenaktiven Stoffen aus 35 bis 40 % Natriumtridecethsulfat und 10 % bis 15 % Natriumlauroamphoacetat und 45 % bis 55 % Wasser besteht.

5. Haarshampoo für krauses und/oder sehr trockenes Haar nach einem der Ansprüche 1 bis 4, **dadurch gekennzeichnet, dass** die Mischung aus oberflächenaktiven Stoffen aus 38 % Natriumtridecethsulfat und 12 % Natriumlauroamphoacetat und 50 % Wasser besteht.

6. Haarshampoo für krauses und/oder sehr trockenes Haar nach einem der Ansprüche 1 bis 5, **dadurch gekennzeichnet, dass** es 20 bis 50 Gew.-% Mischung aus oberflächenaktiven Stoffen mit Bezug auf das Gesamtgewicht des Shampoos umfasst.

7. Haarshampoo für krauses und/oder sehr trockenes Haar nach einem der Ansprüche 1 bis 6, **dadurch gekennzeichnet, dass** die kationische Verbindung, die mindestens eine Verbindung entweder polymerer Art oder oberflächenaktiver Art umfasst, ausgewählt ist aus der Gruppe, bestehend aus: Behentrimoniumchlorid, Polyquaternien, quartäre Ammoniumverbindungen von Alkylketten zwischen C12 und C22, quaternisierte Guarpolymere, quaternisierte Proteine, Cetrimoniumchlorid und dem Komplex, enthaltend Acrylamidopropyltrimoniumchlorid und Acrylamidcopolymer.

8. Haarshampoo für krauses und/oder sehr trockenes Haar nach einem der Ansprüche 1 bis 7, **dadurch gekennzeichnet, dass** die kationische Verbindung, die mindestens eine Verbindung entweder polymerer Art oder oberflächenaktiver Art umfasst, ausgewählt ist aus der Gruppe, bestehen aus: Behentrimoniumchlorid, quaternisiertem Guarpolymer und Certrimoniumchlorid.

9. Haarshampoo für krauses und/oder sehr trockenes Haar nach einem der Ansprüche 1 bis 8, **dadurch gekennzeichnet, dass** es von 0,1 bis 5 Gew.-% kationische Verbindung(en) umfasst, umfassend mindestens eine Verbindung entweder polymerer Art oder oberflächenaktiver Art, mit Bezug auf das Gesamtgewicht des Shampoos.

10. Waschprozess von krausem und/oder sehr trockenem Haar, umfassend die Anwendung eines Haarshampoos für krauses Haar und/oder sehr trockenes Haar nach Anspruch 1 bis 9 auf das Haar.

## Claims

1. Capillary shampoo for frizzy and/or very dry hair comprising:
- 1% to 30% by weight of an oil phase comprising Abyssinian kale oil,
- 10% to 60% by weight of a mixture of surfactants comprising sodium trideceth sulphate and sodium lauroamphoacetate, and
- 0.01% to 10% by weight of a cationic compound comprising at least one compound of either a polymeric type or of a surfactant type.

2. Capillary shampoo for frizzy and/or very dry hair according to claim 1, **characterised in that** the oil phase comprises a combination of Abyssinian kale oil with one or several triglyceride(s) of plant origin.

3. Capillary shampoo for frizzy and/or very dry hair according to claims 1 to 2, **characterised in that** it comprises from 5% to 20% by weight of the oil phase with respect to the total weight of the shampoo.

4. Capillary shampoo for frizzy and/or very dry hair according to one of claims 1 to 3, **characterised in that** the mixture of surfactants is constituted from 35 to 40% of sodium trideceth sulphate and from 10% to 15% of sodium lauroamphoacetate and 45% to 55% water.

5. Capillary shampoo for frizzy and/or very dry hair according to one of claims 1 to 4, **characterised in that** the mixture of surfactants is constituted from 38% sodium trideceth sulphate and 12% sodium lauroamphoacetate and 50% water.

6. Capillary shampoo for frizzy and/or very dry hair according to one of claims 1 to 5, **characterised in that** it comprises from 20% to 50% by weight of a mixture of surfactants with respect to the total weight of the shampoo.

7. Capillary shampoo for frizzy and/or very dry hair according to one of claims 1 to 6, **characterised in that** the cationic compound comprising at least one compound, either or a polymeric nature or of a surfactant nature, is selected from the group comprising: behentrimonium chloride, polyquaterniums, quaternary ammoniums of alkyl chains between C12 and C22, quaternized guar polymers, quaternized proteins, cetrimomium chloride and the complex containing acrylamidopropyltrimonium chloride and acrylamide copolymer.

8. Capillary shampoo for frizzy and/or very dry hair according to one of claims 1 to 7, **characterised in that** the cationic compound comprising at least one compound, either of a polymeric nature or of a surfactant nature, is selected from the group comprising: behentrimonium chloride, quaternized guar polymer and cetrimomium chloride.

9. Capillary shampoo for frizzy and/or very dry hair according to one of claims 1 to 8, **characterised in that** it comprises from 0.1% to 5% by weight of cationic compound(s) comprising at least one compound, either of a polymeric nature or of a surfactant nature, with respect to the total weight of the shampoo.

10. Method for washing frizzy and/or very dry hair comprising the application on the hair of a capillary shampoo for frizzy and/or very dry hair according to claims 1 to 9.
